# EUROPEAN PATENT APPLICATION

(11) **EP 4 600 955 A1**
(43) Date of publication of application: **13.08.2025**
(21) Application number: 23884680.2
(22) Date of filing: 25.10.2023
(51) Int. Cl.: G10L 25/66, A61B 5/08, A61B 5/00

(54) **SIGNAL COLLECTION METHOD, AND ELECTRONIC DEVICE AND STORAGE MEDIUM**

(30) Priority: 01.11.2022 CN 202211357787
(71) Applicant: Huawei Technologies Co., Ltd., Shenzhen, Guangdong 518129 (CN)
(72) Inventor: LI, Jing, Shenzhen, Guangdong 518129 (CN); HUANG, Jiejing, Shenzhen, Guangdong 518129 (CN); WANG, Wei, Shenzhen, Guangdong 518129 (CN); ZHANG, Wei, Shenzhen, Guangdong 518129 (CN)
(74) Representative: MERH-IP Matias Erny Reichl Hoffmann Patentanwälte PartG mbB
(86) International application number: PCT/CN2023/126352
(87) International publication number: WO 2024/093748

(57) **Abstract**

This application provides a signal collection method, an electronic device, and a storage medium. The method includes: collecting an audio signal of a user by using a microphone; performing judging on the collected audio signal of the user, to determine whether the collected audio signal of the user is standard; if the collected audio signal of the user is not standard, determining a reason why the collected audio signal of the user is not standard; and reminding, based on the reason why the collected audio signal of the user is not standard, the user that re-collection is to be performed. According to the method provided in this application, quality of a collected user signal can be improved.

## Description

This application claims priority to Chinese Patent Application No. 202211357787.8, filed with the China National Intellectual Property Administration on November 1, 2022, and entitled "SIGNAL COLLECTION METHOD, ELECTRONIC DEVICE, AND STORAGE MEDIUM", which is incorporated herein by reference in its entirety.

### TECHNICAL FIELD

This application relates to the field of smart wearable devices, and in particular, to a signal collection method, an electronic device, and a storage medium.

### BACKGROUND

Chronic respiratory diseases are an important cause of harm to people's health. Pulmonary function tests are a golden standard for examination of the chronic respiratory diseases, and are of great significance in diagnosis, severity evaluation, disease progression, prognosis and treatment response assessment of the chronic respiratory diseases. Smart wearable devices, for example, smart glasses and smart headsets, are increasingly used for examination of the chronic respiratory diseases due to features such as low costs, ease of carrying, simple operation, and free movement.

However, during use of the foregoing smart wearable devices to examine a chronic respiratory disease of a user, because the smart wearable devices have a high requirement on an audio signal of the user, a non-standard operation of the user, for example, a non-standard blowing action of the user, severely affects quality of a collected signal, and consequently results in false examination of the disease. Therefore, a signal collection method is urgently needed to improve quality of the foregoing collected signal.

### SUMMARY

This application provides a signal collection method, an electronic device, and a storage medium, to improve quality of a collected user signal.

According to a first aspect, this application provides a signal collection method, applied to an electronic device, where the electronic device includes a microphone, and the method includes:
collecting an audio signal of a user by using the microphone;
performing judging on the collected audio signal of the user, to determine whether the collected audio signal of the user is standard;
if the collected audio signal of the user is not standard, determining a reason why the collected audio signal of the user is not standard; and
reminding, based on the reason why the collected audio signal of the user is not standard, the user that re-collection is to be performed.

In this application, the collected audio signal of the user is detected, and when it is determined that the audio signal of the user is not standard, a reason that may cause the signal to be non-standard is analyzed, and the user is reminded to perform adjustment. In this way, a high-quality audio signal can be obtained. This improves accuracy of assessing a health status of the user.

In a possible implementation, before the collecting an audio signal of a user by using the microphone, the method further includes:
turning on the microphone in response to an operation of the user collecting an audio signal.

In this application, signal collection may be started through an active operation of the user, so that autonomy and flexibility of the user can be improved.

In a possible implementation, before the collecting an audio signal of a user by using the microphone, the method further includes:
turning on the microphone in response to a detected change of a physiological parameter of the user.

In this application, the electronic device detects the change of the physiological parameter of the user and starts to collect the audio signal of the user, so that a health status of the user can be detected in a targeted manner, and user experience can be improved.

In a possible implementation, after the collecting an audio signal of a user by using the microphone, the method further includes:
extracting the collected audio signal of the user, to obtain a valid audio segment.

In this application, the valid audio segment is extracted, and the collected audio signal of the user is detected by using the valid audio segment, so that detection efficiency can be improved.

In a possible implementation, the determining a reason why the collected audio signal of the user is not standard includes:
determining, based on a feature of the collected audio signal of the user, the reason why the collected audio signal of the user is not standard.

In this application, the reason why the collected audio signal of the user is not standard is determined based on the feature of the collected audio signal of the user, so that a non-standard action of the user can be simply and effectively checked.

In a possible implementation, the determining, based on a feature of the collected audio signal of the user, the reason why the collected audio signal of the user is not standard includes:
if an amplitude of the valid audio segment is greater than a preset first amplitude threshold, determining that the reason why the collected audio signal of the user is not standard is that a voice of the user is excessively loud;
if an amplitude of the collected audio signal of the user is less than a preset second amplitude threshold, determining that the reason why the collected audio signal of the user is not standard is that a voice of the user is excessively low;
if duration of the valid audio segment is less than a preset duration threshold, determining that the reason why the collected audio signal of the user is not standard is that duration of air blowing performed by the user is excessively short;
if an amplitude of the collected audio signal of the user is greater than a preset third amplitude threshold, determining that the reason why the collected audio signal of the user is not standard is that there is high noise in an ambient environment; or
if a feature of the valid audio segment meets a preset requirement, but an envelope of the valid audio segment does not match an envelope of a standard audio signal, determining that the reason why the collected audio signal of the user is not standard is that a collection pose of the user is incorrect, where
the standard audio signal is an audio signal obtained when collection is performed on the user in a standard pose.

In a possible implementation, the determining a reason why the collected audio signal of the user is not standard includes:
obtaining an image or a video of the user during collection;
identifying a collection operation of the user based on the image or the video; and
determining a difference between the collection operation of the user and a standard collection operation, and determining, based on the difference, the reason why the collected audio signal of the user is not standard.

In this application, a pose of the user in the image or the video is identified by the user during collection, to determine whether a collection action of the user is standard, so that a non-standard action of the user can be simply and effectively checked.

In a possible implementation, the determining a reason why the collected audio signal of the user is not standard includes:
obtaining head action information of the user during collection;
identifying a head action of the user based on the head action information; and
determining a difference between the head action of the user and a standard head action, and determining, based on the difference, the reason why the collected audio signal of the user is not standard.

In this application, the head action of the user is identified by the user during collection, to determine whether a collection action of the user is standard, so that a non-standard action of the user can be simply and effectively checked.

According to a second aspect, this application provides an electronic device, including a processor and a memory. The memory is configured to store a computer program, and the processor is configured to run the computer program, to implement the signal collection method according to the first aspect.

According to a third aspect, this application provides a computer-readable storage medium. The computer-readable storage medium stores a computer program, and when the computer program is run on a computer, the computer is enabled to implement the signal collection method according to the first aspect.

According to a fourth aspect, this application provides a computer program. When the computer program is run on a processor of an electronic device, the electronic device is enabled to perform the signal collection method according to the first aspect.

In a possible design, the program in the fourth aspect may be all or partially stored in a storage medium that is packaged together with a processor, or may be all or partially stored in a memory that is not packaged together with a processor.

### BRIEF DESCRIPTION OF DRAWINGS

FIG. 1 is a diagram of a structure of an electronic device according to an embodiment of this application;
FIG. 2 is a schematic flowchart of an embodiment of a signal collection method according to this application;
FIG. 3a is a diagram of a waveform of an embodiment of a non-standard audio signal according to this application;
FIG. 3b is a diagram of a waveform of another embodiment of a non-standard audio signal according to this application;
FIG. 3c is a diagram of a waveform of still another embodiment of a non-standard audio signal according to this application;
FIG. 3d is a diagram of a waveform of yet another embodiment of a non-standard audio signal according to this application;
FIG. 3e is a diagram of a waveform of still yet another embodiment of a non-standard audio signal according to this application;
FIG. 4 is a schematic flowchart of another embodiment of a signal collection method according to this application; and
FIG. 5 is a diagram of a structure of a signal collection apparatus according to an embodiment of this application.

### DESCRIPTION OF EMBODIMENTS

In embodiments of this application, unless otherwise specified, the character "/" indicates an "or" relationship between associated objects. For example, A/B may indicate A or B. The term "and/or" describes an association relationship between associated objects, and indicates that three relationships may exist. For example, A and/or B may indicate the following three cases: Only A exists, both A and B exist, and only B exists.

It should be noted that, in embodiments of this application, terms such as "first" and "second" are merely intended for distinction in description, and shall not be understood as an indication or implication of relative importance or an implicit indication of a quantity of indicated technical features, or an indication or implication of a sequence.

In embodiments of this application, "at least one" means one or more, and "a plurality of" means two or more. In addition, "at least one of the following items (pieces)" or a similar expression thereof means any combination of these items, including a single item (piece) or any combination of a plurality of items (pieces). For example, at least one item (piece) of A, B, or C may indicate A, B, C, A and B, A and C, B and C, or A, B, and C. Each of A, B, and C may be an element, or may be a set including one or more elements.

In embodiments of this application, "example", "in some embodiments", "in another embodiment", or the like is used to represent giving an example, an illustration, or a description. Any embodiment or design scheme described as an "example" in this application should not be explained as being more preferred or having more advantages than another embodiment or design scheme. Exactly, use of the term "example" is intended to present a concept in a specific manner.

In embodiments of this application, "of (of)", "relevant (corresponding, relevant)", and "corresponding (corresponding)" may sometimes be interchangeably used. It should be noted that meanings expressed by the terms are consistent when differences are not emphasized. In embodiments of this application, communication and transmission may sometimes be interchangeably used. It should be noted that meanings expressed by the terms are consistent when differences are not emphasized. For example, transmission may include sending and/or receiving, and may be a noun or a verb.

In embodiments of this application, "equal to" may be used together with "greater than", and is applicable to a technical solution used when "greater than" is used; or "equal to" may be used together with "less than", and is applicable to a technical solution used when "less than" is used. It should be noted that, when "equal to" is used together with "greater than", "equal to" shall not be used together with "less than"; or when "equal to" is used together with "less than", "equal to" shall not be used together with "greater than".

Chronic respiratory diseases are an important cause of harm to people's health. Pulmonary function tests are a golden standard for examination of the chronic respiratory diseases, and are of great significance in diagnosis, severity evaluation, disease progression, prognosis and treatment response assessment of the chronic respiratory diseases. Smart wearable devices, for example, smart glasses and smart headsets, are increasingly used for examination of the chronic respiratory diseases due to features such as low costs, ease of carrying, simple operation, and free movement.

However, during use of the foregoing smart wearable devices to examine a chronic respiratory disease of a user, because the smart wearable devices have a high requirement on an audio signal of the user, a non-standard operation of the user, for example, a non-standard blowing action of the user, severely affects quality of a collected signal, and consequently results in false examination of the disease. Therefore, a signal collection method is urgently needed to improve quality of the foregoing collected signal.

Based on the foregoing problem, an embodiment of this application provides a signal collection method, applied to an electronic device having a microphone. The microphone may be configured to collect an audio signal of a user through air blowing performed by the user. The electronic device may be a terminal device, for example, a mobile phone, a tablet, or a large screen. The terminal device may also be referred to as user equipment (User Equipment, UE), an access terminal, a subscriber unit, a subscriber station, a mobile station, a remote station, a remote terminal, a mobile device, a user terminal, a terminal, a wireless communication device, a user agent, a user apparatus, or the like. The electronic device may alternatively be a station (STATION, ST) in a WLAN, or may be a cellular phone, a cordless phone, a session initiation protocol (Session Initiation Protocol, SIP) phone, a wireless local loop (Wireless Local Loop, WLL) station, a personal digital assistant (Personal Digital Assistant, PDA) device, a handheld device having a wireless communication function, a computing device or another processing device connected to a wireless modem, a vehicle-mounted device, an internet of vehicles terminal, a computer, a laptop computer, a handheld communication device, a handheld computing device, a satellite radio device, a wireless modem card, a television set top box (set top box, STB), customer premises equipment (customer premises equipment, CPE), and/or another device configured to perform communication in a wireless system, or a mobile terminal in a next-generation communication system like a 5G network, or a mobile terminal in a future evolved public land mobile network (Public Land Mobile Network, PLMN), or the like. The electronic device may alternatively be a wearable device. The wearable device may also be referred to as a smart wearable device, and is a general term for wearable devices that are intelligently designed and developed for daily wear by using a wearable technology, such as glasses, gloves, and a watch. The wearable device is a portable device that can be directly worn on a body or integrated into clothes or an accessory of a user. The wearable device is not merely a hardware device, but further implements a powerful function through software support, data exchange, and cloud interaction.

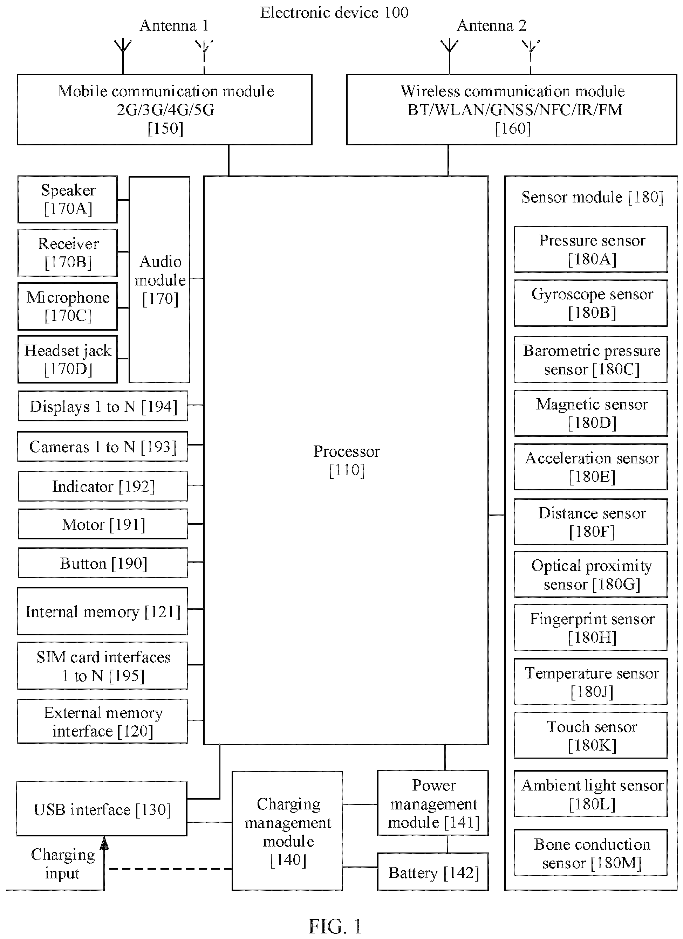

FIG. 1 first shows a diagram of an example of a structure of an electronic device 100.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, or some components may be combined, or some components may be split, or there may be a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent devices, or may be integrated into one or more processors.

The controller may generate an operation control signal based on instruction operation code and a time sequence signal, to complete control of instruction fetch and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a bidirectional synchronous serial bus, including a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be configured to: perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be configured to perform audio communication.

The UART interface is a universal serial data bus, and is configured to perform asynchronous communication. The bus may be a bidirectional communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral device like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement a photographing function of the electronic device 100. The processor 110 communicates with the display 194 through the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB Type-C interface, or the like. The USB interface 130 may be configured to connect to a charger to charge the electronic device 100, or may be configured to transmit data between the electronic device 100 and a peripheral device, or may be configured to connect to a headset for playing audio through a headset. The interface may be further configured to connect to another terminal device, for example, an AR device.

It may be understood that an interface connection relationship between the modules shown in this embodiment of the present invention is merely an example for description, and does not constitute a limitation on a structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. The charging management module 140 may further supply power to the terminal device by using the power management module 141 while charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to: transmit and receive an electromagnetic wave signal. Each antenna in the electronic device 100 may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G, or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit a processed electromagnetic wave to the modem processor for demodulation. The mobile communication module 150 may further amplify a signal modulated by the modem processor, and convert an amplified signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 may implement a display function through the GPU, the display 194, the application processor, and the like. The GPU is a microprocessor for image processing, and is connected to the display 194 and the application processor. The GPU is configured to perform mathematical and geometric computation for graphic rendering. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1.

The electronic device 100 may implement a photographing function through the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, luminance, and complexion of the image. The ISP may further optimize parameters such as exposure and a color temperature of a shooting scene. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transform and the like on frequency energy.

The video codec is configured to compress or decompress a digital video. The electronic device 100 may support one or more video codecs. In this way, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor. The NPU quickly processes input information with reference to a structure of a biological neural network, for example, a transfer mode between human brain neurons, and may further continuously perform self-learning. The NPU may be used to implement applications such as intelligent cognition of the electronic device 100, for example, image recognition, facial recognition, voice recognition, and text understanding.

The external memory interface 120 may be configured to connect to an external memory card, for example, a micro SD card, to extend a storage capability of the electronic device 100. The external memory card communicates with the processor 110 through the external memory interface 120, to implement a data storage function. For example, files such as music and videos are stored in the external memory card.

The internal memory 121 may be configured to store computer-executable program code. The executable program code includes instructions. The internal memory 121 may include a program storage area and a data storage area. The program storage area may store an operating system, an application required by at least one function (for example, a voice playing function or an image playing function), and the like. The data storage area may store data (for example, audio data and an address book) and the like that are created during use of the electronic device 100. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications of the electronic device 100 and data processing.

The electronic device 100 may implement an audio function, for example, music playing or recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or voice information is received through the electronic device 100, the receiver 170B may be put close to a human ear to listen to a voice.

The microphone 170C, also referred to as a "mike" or a "mic", is configured to convert a sound signal into an electrical signal. When making a call or sending voice information, a user may make a sound by moving a human mouth close to the microphone 170C, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, thereby implementing a directional recording function and the like.

The headset jack 170D is configured to connect to a wired headset. The headset jack 170D may be the USB interface 130, or may be a 3.5 mm open mobile terminal device platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change of the capacitance. When a touch operation is performed on the display 194, the electronic device 100 detects intensity of the touch operation by using the pressure sensor 180A. The electronic device 100 may also calculate a touch location based on a detection signal of the pressure sensor 180A. In some embodiments, touch operations that are performed at a same touch location but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyroscope sensor 180B may be configured to determine a moving posture of the electronic device 100. In some embodiments, an angular velocity of the electronic device 100 around three axes (namely, axes x, y, and z) may be determined by using the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude based on a barometric pressure value measured by the barometric pressure sensor 180C, to assist in positioning and navigation.

The magnetic sensor 180D includes a Hall sensor. The electronic device 100 may detect opening and closing of a flip cover or a leather case by using the magnetic sensor 180D. In some embodiments, when the electronic device 100 is a flip phone, the electronic device 100 may detect opening and closing of a flip cover by using the magnetic sensor 180D. Further, a feature like automatic unlocking of the flip cover is set based on a detected opening or closing state of the leather case or a detected opening or closing state of the flip cover.

The acceleration sensor 180E may detect accelerations in various directions (usually on three axes) of the electronic device 100. A magnitude and a direction of gravity may be detected when the electronic device 100 is still. The acceleration sensor 180E may be further configured to identify a posture of the terminal device, and is used in an application like switching between a landscape mode and a portrait mode or a pedometer.

The distance sensor 180F is configured to measure a distance. The electronic device 100 may measure the distance in an infrared manner or a laser manner. In some embodiments, in a shooting scene, the electronic device 100 may measure a distance by using the distance sensor 180F to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that a user holds the electronic device 100 close to an ear for a call, to automatically turn off a screen to save power. The optical proximity sensor 180G may also be used in a leather case mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to avoid abnormal shutdown of the electronic device 100 caused by a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a "touch control device". The touch sensor 180K may be disposed on the display 194, and the touch sensor 180K and the display 194 constitute a touchscreen that is also referred to as a "touch screen". The touch sensor 180K is configured to detect a touch operation performed on or near the touch sensor. The touch sensor may transfer the detected touch operation to the application processor, to determine a type of the touch event. A visual output related to the touch operation may be provided by using the display 194. In some other embodiments, the touch sensor 180K may alternatively be disposed on a surface of the electronic device 100 at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may alternatively be disposed in a headset to be combined into a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, or the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effect. The motor 191 may also correspond to different vibration feedback effect for touch operations performed on different areas of the display 194. Different application scenarios (for example, a time reminder, information receiving, an alarm clock, and a game) may also correspond to different vibration feedback effect. Touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 may also be compatible with an external memory card. The electronic device 100 interacts with a network by using the SIM card, to implement functions such as conversation and data communication. In some embodiments, the electronic device 100 uses an eSIM, namely, an embedded SIM card. The eSIM card may be embedded into the electronic device 100, and cannot be separated from the electronic device 100.

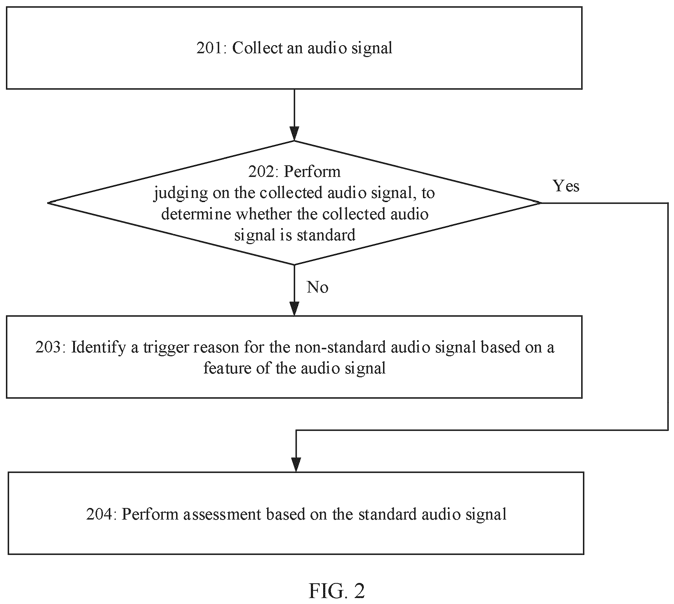

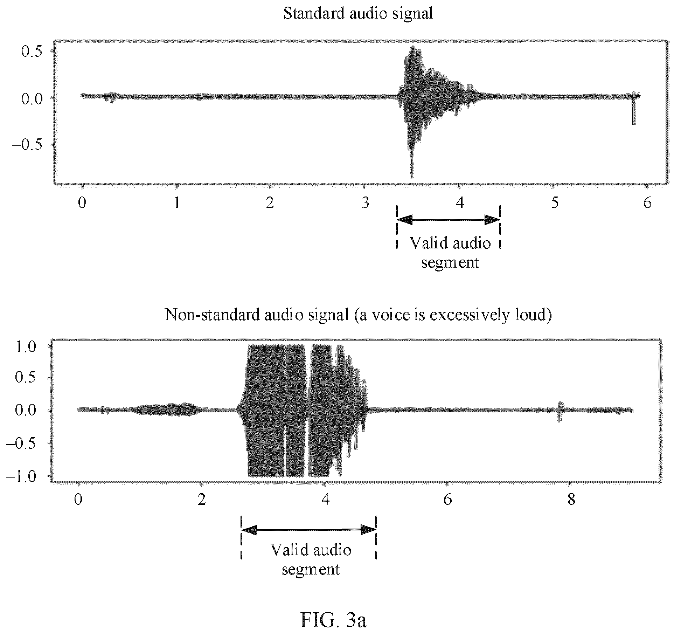

The signal collection method provided in embodiments of this application is described herein with reference to FIG. 2 and FIG. 3a to FIG. 3e. The foregoing signal collection method may be applied to an electronic device. A type of the electronic device is not specially limited in embodiments of this application.

FIG. 2 is a schematic flowchart of an embodiment of a signal collection method according to this application. The method specifically includes the following steps.

Step 201: Collect an audio signal.

Specifically, the audio may be audio of a respiratory sound of a user. That is, a chronic respiratory disease of the user may be assessed by detecting the respiratory sound of the user.

The user may actively trigger collection of the audio signal. For example, the user may perform an operation on an electronic device, to start assessment on the chronic respiratory disease of the user. In this case, a microphone of the electronic device is in an on state, and is configured to collect the audio signal of the user.

In some optional embodiments, the electronic device may further prompt, based on a change of a physiological parameter of the user, the user to perform assessment on the chronic respiratory disease. The physiological parameter of the user, for example, breathing, blood oxygen, and heart rate variability (Heart Rate Variability, HRV), may be collected by using a sensor of the electronic device. For example, the electronic device may continuously monitor the change of the physiological parameter of the user. When detecting that a change range of the physiological parameter of the user exceeds a preset range, the electronic device may prompt the user to perform assessment on the chronic respiratory disease. In this case, a microphone of the electronic device is in an on state, and is configured to collect the audio signal of the user.

A manner of triggering collection of the audio signal is not specially limited in this application.

Step 202: Perform judging on the collected audio signal, to determine whether the collected audio signal is standard.

Specifically, the electronic device may collect the audio signal of the user through air blowing performed by the user.

It may be understood that, after the user finishes air blowing, the electronic device may collect a segment of audio signal of the user, and therefore may perform judging on the segment of audio signal of the user, to determine whether the segment of audio signal of the user is standard, or whether the segment of audio signal of the user matches a standard audio signal.

Because whether an air blowing action of the user is standard directly affects an assessment result of the disease, the electronic device has a high requirement on the collected audio signal. For example, whether a placement manner of the electronic device is standard when the user blows air, and whether the blowing action of the user is standard affect the assessment result. Therefore, a standard audio signal may be pre-stored in the electronic device, and the standard audio signal may be an audio signal obtained by the user through a standard blowing action. For example, the standard audio signal has information such as standard air blowing duration, a standard volume, and a standard peak envelope. Therefore, the collected audio signal of the user may be compared with the standard audio signal, to determine whether the collected audio signal of the user is a standard audio signal.

In some optional embodiments, an audio judging model may alternatively be preestablished in the electronic device. The audio judging model may be a deep learning model. For example, the audio judging model may be trained by using the standard audio signal and various interference signals. Therefore, the collected audio signal of the user may be determined, to determine whether the collected audio signal of the user is a standard audio signal.

A manner of identifying the collected audio signal of the user is not specially limited in embodiments of this application.

If it is determined that the collected audio signal of the user is a standard audio signal, step 204 may be further performed.

If it is determined that the collected audio signal of the user is a non-standard audio signal, step 203 may be further performed.

In some optional embodiments, before identification of the collected audio signal of the user, to improve identification efficiency and accuracy, a valid audio segment may be further extracted from the collected audio signal of the user. The valid audio segment may represent an audio segment with an obvious audio feature of the user. The valid audio segment may be determined based on an audio amplitude. For example, a standard audio amplitude may be preset, and an audio segment near the standard audio amplitude may be used as the valid audio segment.

It may be understood that, during identification of the valid audio segment, a start location and an end location of the valid audio segment may be marked in the entire collected audio signal of the user, and the marked valid audio segment may be processed; or the valid audio segment is captured from the entire collected audio signal of the user, and the valid audio segment is processed. This is not specially limited in embodiments of this application.

Step 203: Identify a trigger reason for the non-standard audio signal.

Specifically, during identification of the trigger reason for the non-standard audio signal, identification may be performed according to the following several scenarios:
Scenario 1: A voice is excessively loud.

In the foregoing scenario 1, if the voice of the user is excessively loud, in terms of an audio feature, the audio amplitude of the valid audio segment may exceed a preset amplitude threshold, or the audio amplitude of the valid audio segment is at a high level for a period of time. A main reason may be that the user is excessively close to the microphone of the electronic device during air blowing. The preset amplitude threshold may be a value greater than a maximum amplitude in the standard audio signal. For ease of description, the preset amplitude threshold is referred to as a preset first amplitude threshold in this specification. When the electronic device identifies the trigger reason for the non-standard audio signal based on the collected audio signal of the user, if it is detected that the audio amplitude of the collected audio signal of the user exceeds a range, or the audio amplitude of the collected audio signal of the user is at a high level for a period of time, it may be determined that the voice of the user is excessively loud when the audio signal is collected. That is, the trigger reason for the non-standard audio signal is that the voice of the user is excessively loud. In this case, the user may be reminded to keep an appropriate distance from the electronic device before collection of an audio signal, so that the electronic device can collect a higher-quality audio signal that meets a requirement of the standard audio signal.

FIG. 3a is a diagram of comparison between the audio signal of the user and the standard audio signal in scenario 1. Refer to FIG. 3a. The upper figure is a waveform diagram of the standard audio signal, and the lower figure is a waveform diagram of the collected audio signal of the user. It may be learned from the lower figure that an amplitude of the valid audio segment in the collected audio signal of the user is far greater than an amplitude of a valid audio segment in the standard audio signal. Therefore, it may be determined that the problem may be caused by the excessively loud voice of the user.

Scenario 2: A voice is excessively low.

In the foregoing scenario 2, if the voice of the user is excessively low, in terms of an audio feature, an amplitude of the collected audio signal of the user may be at a low level for a period of time (for example, less than a small preset amplitude threshold, for ease of description, the preset amplitude threshold is referred to as a preset second amplitude threshold in this specification). A main reason may be that the user is excessively far away from the microphone of the electronic device during air blowing, or collection of an audio signal is not performed on the user. When the electronic device identifies the trigger reason for the non-standard audio signal based on the collected audio signal of the user, if it is detected that the amplitude of the collected audio signal of the user is at a low level for a period of time, it may be determined that the voice of the user is excessively low when the audio signal is collected. That is, the trigger reason for the non-standard audio signal is that the voice of the user is excessively low. In this case, the user may be reminded to keep an appropriate distance from the electronic device before collection of an audio signal, or the user may be reminded that collection of an audio signal starts, so that the electronic device can collect a higher-quality audio signal that meets a requirement of the standard audio signal.

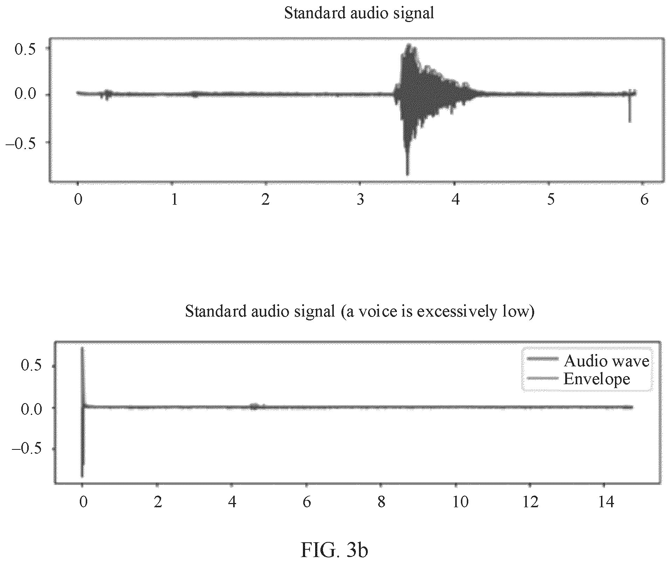

FIG. 3b is a diagram of comparison between the audio signal of the user and the standard audio signal in scenario 2. Refer to FIG. 3b. The upper figure is a waveform diagram of the standard audio signal, and the lower figure is a waveform diagram of the collected audio signal of the user. It may be learned from the lower figure that the amplitude of the collected audio signal of the user is at a low level for a period of time, and is almost close to 0. Therefore, it may be determined that the problem may be caused by the excessively low voice of the user.

Scenario 3: Duration is excessively short.

In the foregoing scenario 3, if continuous air blowing duration of the user is excessively short or the user terminates audio collection in advance in an audio collection process, for example, the continuous air blowing duration of the user is less than a preset duration threshold, a requirement on audio signal collection may not be met, and in terms of an audio feature, it may be represented as that duration of the valid audio segment is less than the preset duration threshold. When the electronic device identifies the trigger reason for the non-standard audio signal based on the collected audio signal of the user, if it is detected that duration of the collected audio signal of the user is less than the preset duration threshold, it may be determined that duration of collecting the audio signal by the user is excessively short. That is, the trigger reason for the non-standard audio signal is that duration of air blowing performed by the user is excessively short. In this case, the user may be reminded that collection of an audio signal needs to be performed based on a duration requirement, so that the electronic device can collect a higher-quality audio signal that meets a requirement of the standard audio signal.

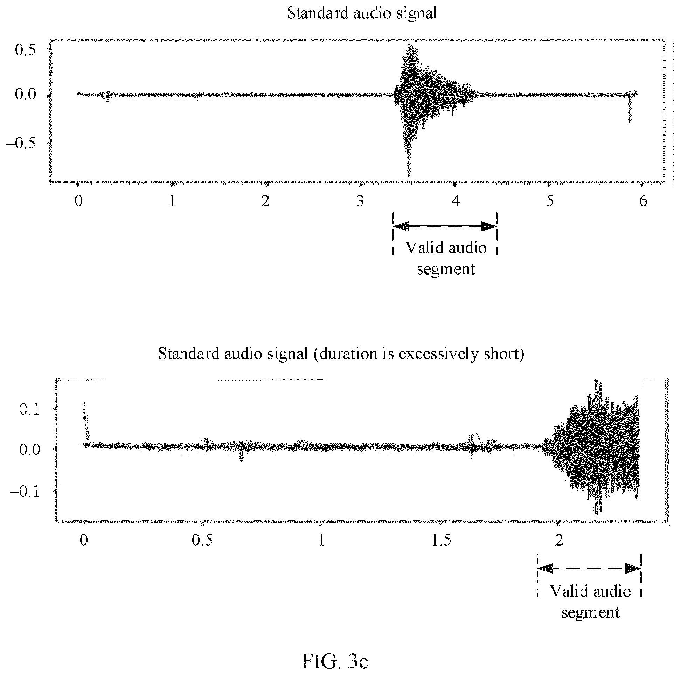

FIG. 3c is a diagram of comparison between the audio signal of the user and the standard audio signal in scenario 3. Refer to FIG. 3c. The upper figure is a waveform diagram of the standard audio signal, and the lower figure is a waveform diagram of the collected audio signal of the user. It may be learned from the lower figure that the duration of the valid audio segment in the collected audio signal of the user is less than duration of the standard audio signal. Therefore, it may be determined that the problem may be caused by the excessively short duration of air blowing performed by the user.

Scenario 4: Noise is excessively high.

In the foregoing scenario 4, if ambient noise is excessively high, in terms of an audio feature, it may be represented as that an amplitude of the entire collected audio signal of the user is at a high level for a period of time (for example, greater than a large preset amplitude threshold, and for ease of description, the preset amplitude threshold is referred to as a preset third amplitude threshold in this specification). A main reason may be that noise in an ambient environment is high, and the audio signal of the user is drowned in the noise. When the electronic device identifies the trigger reason for the non-standard audio signal based on the collected audio signal of the user, if it is detected that the amplitude of the collected audio signal of the user is at a high level for a period of time, it may be determined that the noise in the ambient environment is high. That is, the trigger reason for the non-standard audio signal is that the noise in the ambient environment is high. In this case, the user may be reminded that collection of an audio signal needs to be performed in a quiet environment, so that the electronic device can collect a higher-quality audio signal that meets a requirement of the standard audio signal.

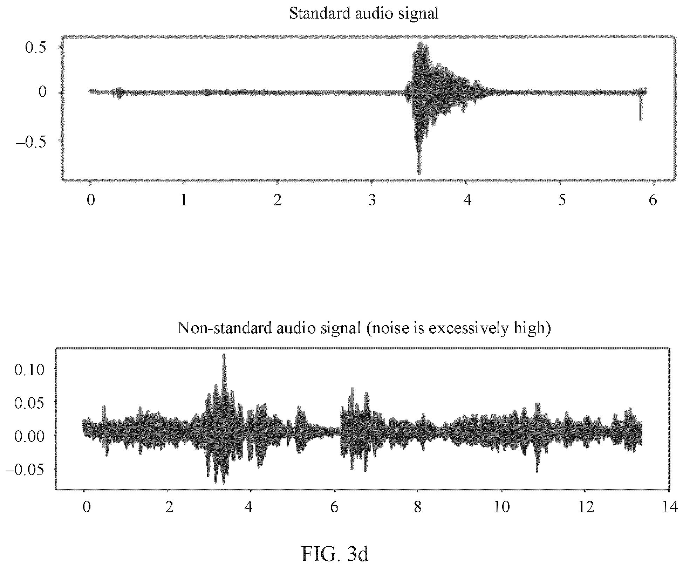

FIG. 3d is a diagram of comparison between the audio signal of the user and the standard audio signal in scenario 4. Refer to FIG. 3d. The upper figure is a waveform diagram of the standard audio signal, and the lower figure is a waveform diagram of the collected audio signal of the user. It may be learned from the lower figure that the amplitude of the entire collected audio signal of the user is at a high level, for example, is far greater than an amplitude of the standard audio signal. Therefore, it may be determined that the problem may be caused by the excessively high noise in the ambient environment.

### Scenario 5: Other scenarios.

In the foregoing scenario 5, the valid audio segment may be detected. If it is determined, after the valid audio segment is detected, that a scenario is not any one of the foregoing scenario 1 to scenario 4, it may be considered that the user is in the scenario 5. That is, except for the reasons that cause the non-standard audio signal in the foregoing scenario 1 to scenario 4, scenarios corresponding to other reasons may be all classified as scenario 5. In the foregoing scenario 5, it may be considered that the non-standard audio signal is generated because a collection pose of the user is incorrect. In terms of an audio feature, it may be represented as that there is no obvious feature. For example, an amplitude is not excessively high or excessively low, and duration of the audio signal is normal. However, an envelope of the valid audio segment does not match an envelope of the standard audio signal. Therefore, it may be determined that the non-standard audio signal is generated because the collection pose of the user is incorrect. When determining that the non-standard audio signal is caused by the incorrect collection pose of the user, the electronic device may remind the user to change the pose for re-collection, so that the electronic device can collect a higher-quality audio signal that meets a requirement of the standard audio signal.

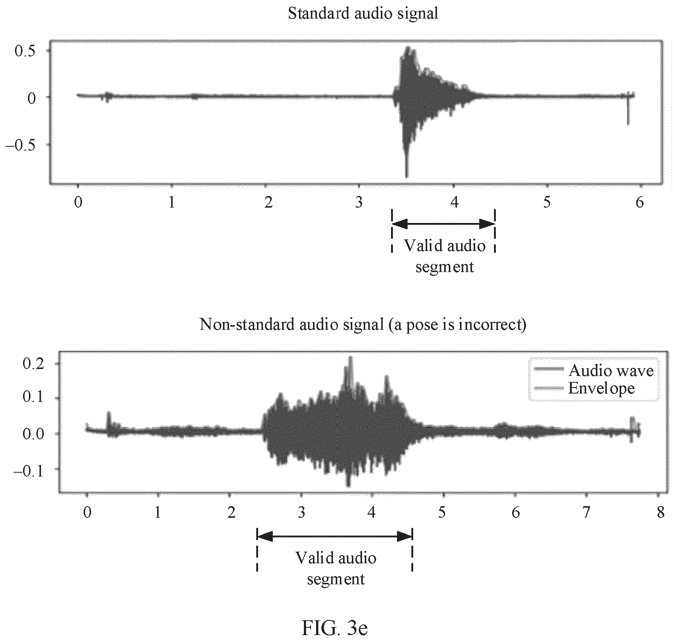

FIG. 3e is a diagram of comparison between the audio signal of the user and the standard audio signal in scenario 5. Refer to FIG. 3e. The upper figure is a waveform diagram of the standard audio signal, and the lower figure is a waveform diagram of the collected audio signal of the user. It may be learned from the lower figure that both the duration and the amplitude of the valid audio segment in the collected audio signal of the user meet requirements, but the envelope of the valid audio segment does not match the envelope of the standard audio signal. Therefore, it may be determined that the problem is caused by an incorrect air blowing pose of the user.

Step 204: Perform assessment based on the standard audio signal.

Specifically, if it is determined that the collected audio signal of the user is a standard audio signal, a health status of the user may be assessed based on the standard audio signal. An assessment manner may be based on the conventional technology, and a specific assessment manner is not described herein. For example, an assessment model may be preset in the electronic device. After obtaining the standard audio signal, the electronic device may input the standard audio signal into the preset assessment model, so that an assessment result may be obtained.

In this embodiment of this application, the collected audio signal of the user is detected, and when it is determined that the audio signal of the user is not standard, a reason that may cause the signal to be non-standard is analyzed, and the user is reminded to perform adjustment. In this way, a high-quality audio signal can be obtained. This improves accuracy of assessing a health status of the user.

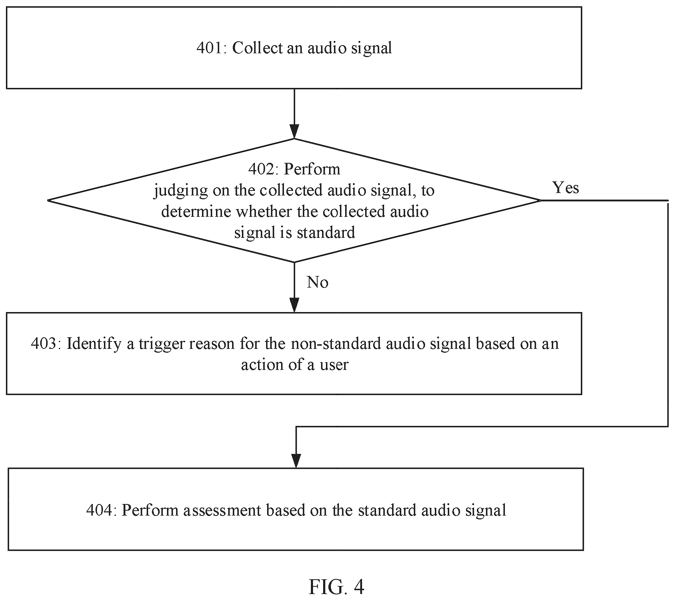

FIG. 4 is a schematic flowchart of another embodiment of a signal collection method according to this application. The method specifically includes the following steps.

Step 401: Collect an audio signal.

Specifically, for a specific implementation of step 401, refer to related descriptions of step 201. Details are not described herein again.

Step 402: Perform judging on the collected audio signal, to determine whether the collected audio signal is standard.

Specifically, for a specific implementation of step 402, refer to related descriptions of step 202. Details are not described herein again.

If it is determined that the collected audio signal of a user is a standard audio signal, step 304 may be further performed.

If it is determined that the collected audio signal of a user is a non-standard audio signal, step 303 may be further performed.

Step 403: Identify a trigger reason for the non-standard audio signal based on an action of the user.

Specifically, a manner of identifying the trigger reason for the non-standard audio signal based on the action of the user may include the following two manners:

### Manner 1: Image detection

In the manner 1, an electronic device may further capture an image or a video of the user during collection of the audio signal of the user. An example in which the electronic device is a device having an image shooting apparatus is used. For example, the electronic device may be a mobile phone, a tablet, a smartwatch, or a large screen. The user may point a camera at the user during air blowing, to allow the camera to perform image shooting on a pose of the user in the form of an image or a video. This is not specially limited in embodiments of this application. For example, if an image is obtained by image shooting, when it is determined that the collected audio signal of the user is a standard audio signal, identification may be performed on a pose of the user in the image. Then, the identified pose of the user may be compared with a standard pose for audio signal collection, to determine a difference between the identified pose of the user and the standard pose for audio signal collection. In addition, after the difference is determined, the user may be reminded of the difference, so that the user adjusts the pose to collect a higher-quality audio signal.

If a video is obtained by image shooting, when it is determined that the collected audio signal of the user is a standard audio signal, one or more video frames in the video may be captured, and identification may be performed on a pose of the user in the one or more video frames. Then, the identified pose of the user may be compared with a standard pose for audio signal collection, to determine a difference between the identified pose of the user and the standard pose for audio signal collection. In addition, after the difference between the poses is determined, the user may be reminded of the difference between the poses, so that the user adjusts the pose to collect a higher-quality audio signal.

### Manner 2: Action identification

In the manner 2, an electronic device may further collect head action information of the user during collection of the audio signal of the user. An example in which the electronic device is a device having a motion sensor is used. For example, the electronic device may be smart glasses or a smart headset. When the user blows air, the electronic device may collect the head action information of the user by using the motion sensor. When it is determined that the collected audio signal of the user is a standard audio signal, identification may be performed based on the collected head action information of the user, to determine a head action of the user, and the identified head action of the user is compared with a standard head action for audio signal collection. For example, the standard head action may include deep breathing, whether duration of air blowing is sufficient, and the like, to determine a difference between the identified head action of the user and the standard head action for audio signal collection. In addition, after the difference between the head actions is determined, the user may be reminded of the difference between the head actions, so that the user adjusts the head action to collect a higher-quality audio signal.

Step 404: Perform assessment based on the standard audio signal.

Specifically, for a specific implementation of step 404, refer to related descriptions of step 204. Details are not described herein again.

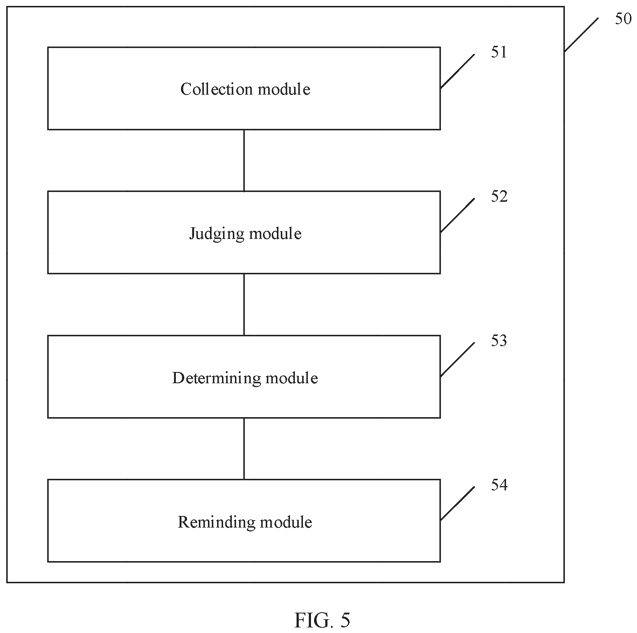

FIG. 5 is a diagram of a structure of an embodiment of a signal collection apparatus according to this application. As shown in FIG. 5, the signal collection apparatus 50 is used in an electronic device, the electronic device includes a microphone, and the signal collection apparatus 50 may include a collection module 51, a judging module 52, a determining module 53, and a reminding module 54.

The collection module 51 is configured to collect an audio signal of a user by using the microphone.

The judging module 52 is configured to perform judging on the collected audio signal of the user, to determine whether the collected audio signal of the user is standard.

The determining module 53 is configured to: if the collected audio signal of the user is not standard, determine a reason why the collected audio signal of the user is not standard.

The reminding module 54 is configured to remind, based on the reason why the collected audio signal of the user is not standard, the user that re-collection is to be performed.

In a possible implementation, the signal collection apparatus 50 may further include:
a turn-on module, configured to turn on the microphone in response to an operation of the user collecting an audio signal.

In a possible implementation, the turn-on module may be configured to turn on the microphone in response to a detected change of a physiological parameter of the user.

In a possible implementation, the signal collection apparatus 50 may further include:
an extraction module, configured to extract the collected audio signal of the user, to obtain a valid audio segment.

In a possible implementation, the determining module is specifically configured to determine, based on a feature of the collected audio signal of the user, the reason why the collected audio signal of the user is not standard.

In a possible implementation, the determining module is specifically configured to: if an amplitude of the valid audio segment is greater than a preset first amplitude threshold, determine that the reason why the collected audio signal of the user is not standard is that a voice of the user is excessively loud;
if an amplitude of the collected audio signal of the user is less than a preset second amplitude threshold, determine that the reason why the collected audio signal of the user is not standard is that a voice of the user is excessively low;
if duration of the valid audio segment is less than a preset duration threshold, determine that the reason why the collected audio signal of the user is not standard is that duration of air blowing performed by the user is excessively short;
if an amplitude of the collected audio signal of the user is greater than a preset third amplitude threshold, determine that the reason why the collected audio signal of the user is not standard is that there is high noise in an ambient environment; or
if a feature of the valid audio segment meets a preset requirement, but an envelope of the valid audio segment does not match an envelope of a standard audio signal, determine that the reason why the collected audio signal of the user is not standard is that a collection pose of the user is incorrect, where
the standard audio signal is an audio signal obtained when collection is performed on the user in a standard pose.

In a possible implementation, the determining module is specifically configured to: obtain an image or a video of the user during collection;
identify a collection operation of the user based on the image or the video; and
determine a difference between the collection operation of the user and a standard collection operation, and determine, based on the difference, the reason why the collected audio signal of the user is not standard.

In a possible implementation, the determining module is specifically configured to: obtain head action information of the user during collection;
identify a head action of the user based on the head action information; and
determine a difference between the head action of the user and a standard head action,
and determine, based on the difference, the reason why the collected audio signal of the user is not standard.

The signal collection apparatus 50 provided in the embodiment shown in FIG. 5 may be configured to perform the technical solutions in method embodiments in this application. For an implementation principle and technical effects of the signal collection apparatus 50, further refer to related descriptions in the method embodiments.

It should be understood that division into the modules of the signal collection apparatus 50 shown in FIG. 5 is merely logical function division. In actual implementation, all or some of the modules may be integrated into one physical entity, or the modules may be physically separated. In addition, all of the modules may be implemented in a form of software invoked by a processing element, or may be implemented in a form of hardware; or some modules may be implemented in a form of software invoked by a processing element, and some modules may be implemented in a form of hardware. For example, a detection module may be a separately-disposed processing element, or may be integrated into a chip of the electronic device for implementation. Implementations of other modules are similar to the implementation of the detection module. In addition, all or some of the modules may be integrated together, or may be implemented independently. In an implementation process, steps in the foregoing methods or the foregoing modules can be implemented by using a hardware integrated logic circuit in the processor element, or by using instructions in a form of software.

For example, the foregoing modules may be one or more integrated circuits configured to implement the foregoing methods, for example, one or more application-specific integrated circuits (Application-Specific Integrated Circuit, ASIC for short below), or one or more microprocessors (Digital Signal Processor, DSP for short below), or one or more field programmable gate arrays (Field Programmable Gate Array, FPGA for short below). For another example, the modules may be integrated together and implemented in a form of a system-on-a-chip (System-On-a-Chip, SOC for short below).

In the foregoing embodiments, the processor may include, for example, a CPU, a DSP, a microcontroller, or a digital signal processor, and may further include a GPU, an embedded neural-network processing unit (Neural-network Processing Unit, NPU for short below), and an image signal processor (Image Signal Processor, ISP for short below). The processor may further include a necessary hardware accelerator or a logic processing hardware circuit, for example, an ASIC, or one or more integrated circuits configured to control program execution of the technical solutions in this application. In addition, the processor may have a function of operating one or more software programs. The software program may be stored in a storage medium.

An embodiment of this application further provides a computer-readable storage medium. The computer-readable storage medium stores a computer program. When the computer program is run on a computer, the computer is enabled to perform the methods provided in embodiments of this application.

An embodiment of this application further provides a computer program product. The computer program product includes a computer program. When the computer program is run on a computer, the computer is enabled to perform the methods provided in embodiments of this application.

In embodiments of this application, "at least one" means one or more, and "a plurality of" means two or more. A term "and/or" describes an association relationship between associated objects and indicates that three relationships may exist. For example, A and/or B may indicate the following cases: Only A exists, both A and B exist, and only B exists, where A and B may be singular or plural. The character "/" generally indicates an "or" relationship between the associated objects. "At least one of the following items" and a similar expression thereof mean any combination of these items, including a single item or any combination of a plurality of items. For example, at least one of a, b, and c may indicate a, b, c, a and b, a and c, b and c, or a, b, and c, where a, b, and c may be singular or plural.

A person of ordinary skill in the art may be aware that units and algorithm steps described in embodiments disclosed in this specification can be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In several embodiments provided in this application, when any of the functions is implemented in a form of a software functional unit and sold or used as an independent product, the function may be stored in a computer-readable storage medium. Based on such an understanding, the technical solutions of this application essentially, or the part contributing to the conventional technology, or some of the technical solutions may be implemented in a form of a software product. The computer software product is stored in a storage medium, and includes several instructions for instructing a computer device (which may be a personal computer, a server, a network device, or the like) to perform all or some of the steps of the methods described in embodiments of this application. The foregoing storage medium includes any medium that can store program code, such as a USB flash drive, a removable hard disk, a read-only memory (Read-Only Memory, ROM for short below), a random access memory (Random Access Memory, RAM for short below), a magnetic disk, or an optical disc.

The foregoing descriptions are merely specific implementations of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A signal collection method, applied to an electronic device, wherein the electronic device comprises a microphone, and the method comprises:
collecting an audio signal of a user by using the microphone;
performing judging on the collected audio signal of the user, to determine whether the collected audio signal of the user is standard;
if the collected audio signal of the user is not standard, determining a reason why the collected audio signal of the user is not standard; and
reminding, based on the reason why the collected audio signal of the user is not standard, the user that re-collection is to be performed.

2. The method according to claim 1, wherein before the collecting an audio signal of a user by using the microphone, the method further comprises:
turning on the microphone in response to an operation of the user collecting an audio signal.

3. The method according to claim 1, wherein before the collecting an audio signal of a user by using the microphone, the method further comprises:
turning on the microphone in response to a detected change of a physiological parameter of the user.

4. The method according to any one of claims 1 to 3, wherein after the collecting an audio signal of a user by using the microphone, the method further comprises:
extracting the collected audio signal of the user, to obtain a valid audio segment.

5. The method according to any one of claims 1 to 4, wherein the determining a reason why the collected audio signal of the user is not standard comprises:
determining, based on a feature of the collected audio signal of the user, the reason why the collected audio signal of the user is not standard.

6. The method according to claim 5, wherein the determining, based on a feature of the collected audio signal of the user, the reason why the collected audio signal of the user is not standard comprises:
if an amplitude of the valid audio segment is greater than a preset first amplitude threshold, determining that the reason why the collected audio signal of the user is not standard is that a voice of the user is excessively loud;
if an amplitude of the collected audio signal of the user is less than a preset second amplitude threshold, determining that the reason why the collected audio signal of the user is not standard is that a voice of the user is excessively low;
if duration of the valid audio segment is less than a preset duration threshold, determining that the reason why the collected audio signal of the user is not standard is that duration of air blowing performed by the user is excessively short;
if an amplitude of the collected audio signal of the user is greater than a preset third amplitude threshold, determining that the reason why the collected audio signal of the user is not standard is that there is high noise in an ambient environment; or
if a feature of the valid audio segment meets a preset requirement, but an envelope of the valid audio segment does not match an envelope of a standard audio signal, determining that the reason why the collected audio signal of the user is not standard is that a collection pose of the user is incorrect, wherein
the standard audio signal is an audio signal obtained when collection is performed on the user in a standard pose.

7. The method according to any one of claims 1 to 4, wherein the determining a reason why the collected audio signal of the user is not standard comprises:
obtaining an image or a video of the user during collection;
identifying a collection operation of the user based on the image or the video; and
determining a difference between the collection operation of the user and a standard collection operation, and determining, based on the difference, the reason why the collected audio signal of the user is not standard.

8. The method according to any one of claims 1 to 4, wherein the determining a reason why the collected audio signal of the user is not standard comprises:
obtaining head action information of the user during collection;
identifying a head action of the user based on the head action information; and
determining a difference between the head action of the user and a standard head action, and determining, based on the difference, the reason why the collected audio signal of the user is not standard.

9. An electronic device, comprising a processor and a memory, wherein the memory is configured to store a computer program, and the processor is configured to run the computer program, to implement the signal collection method according to any one of claims 1 to 8.

10. A computer-readable storage medium, wherein the computer-readable storage medium stores a computer program, and when the computer program is run on a computer, the signal collection method according to any one of claims 1 to 8 is implemented.
